(19) Europäisches Patentamt
European Patent Office
Office européen des brevets

(11) Veröffentlichungsnummer: **0 432 406 B1**

(12) **EUROPÄISCHE PATENTSCHRIFT**

(45) Veröffentlichungstag der Patentschrift: **20.07.94**

(51) Int. Cl.$^5$: **C07C 327/26**

(21) Anmeldenummer: **90119702.0**

(22) Anmeldetag: **15.10.90**

# (54) Polysulfidderivate, Verfahren zu ihrer Herstellung und Verwendung zur Vernetzung von Natur- und Synthesekautschuken.

(30) Priorität: **12.12.89 DE 3941002**

(43) Veröffentlichungstag der Anmeldung:
**19.06.91 Patentblatt 91/25**

(45) Bekanntmachung des Hinweises auf die Patenterteilung:
**20.07.94 Patentblatt 94/29**

(84) Benannte Vertragsstaaten:
**AT BE CH DE ES FR GB IT LI LU NL SE**

(56) Entgegenhaltungen:
**DE-C- 2 265 382**
**FR-A- 2 165 867**

(73) Patentinhaber: **HÜLS AKTIENGESELLSCHAFT**

**D-45764 Marl(DE)**

(72) Erfinder: **Hörpel, Gerhard, Dr.**
**Lerchenhain 84**
**W-4405 Nottuln(DE)**
Erfinder: **Nordsiek, Karl-Heinz, Dr.**
**Neumarkstrasse 4**
**W-4370 Marl(DE)**

**EP 0 432 406 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Sulfidderivate aromatischer Dithiocarbonsäuren, ein Verfahren zu ihrer Herstellung und ihre Verwendung als nitrosaminfreie Vernetzungsmittel von Kautschuken zur Verbesserung der Reversionsstabilität. Die erfindungsgemäßen Polysulfidderivate haben die allgemeine Formel

$(A - S - S -)_z X - S - S - A'$

Die Vernetzung von Kautschuk mit Schwefel oder Schwefelspendern und Beschleunigersystemen liefert in der Regel Vulkanisate, deren Polymerketten in der Anfangsphase durch polysulfidische Schwefelnetzbrükken verknüpft sind. Polysulfidisch vernetzte Vulkanisate führen bei einer Vielzahl von Eigenschaften zu optimalen Ergebnissen (vgl. A. V. Chapman, M. Porter "Sulfur Vulkanisation Chemistry" in "Natural Rubber Science and Technology", A. D. Roberts, ed., Oxford Press 1988). Dies trifft neben der mechanischen Festigkeit insbesondere auf den Weiterreißwiderstand als auch auf den Abriebwiderstand zu. Ein Nachteil liegt jedoch in der Reversion solcher schwefelhaltigen Systeme. Unter dem Begriff Reversion faßt man den Wiederabfall der Vulkanisateigenschaften einerseits während des Vulkanisationsprozesses und andererseits die anaerobe Alterung bei dynamischer Beanspruchung zusammen. Die Vorstellungen zum Mechanismus gehen dahin, daß unter thermischer Belastung ein Abbau der polysulfidischen Brücken bis hin zur monosulfidischen Anordnung stattfindet. Der dabei freiwerdende Schwefel lagert sich einerseits an Polymersegmente an und erhöht damit deren Glastemperatur $T_g$ und katalysiert zum anderen den Angriff des Sauerstoffes auf das Vulkanisat. Beide Effekte führen zu einer Schädigung des Netzwerks. Die genannten Folgereaktionen des Schwefels, die in Abhängigkeit von Reaktionszeit und -temperatur bereits im Verlauf des Vulkanisationsprozesses einsetzen, beeinflussen wesentlich die Leistungsgrenzen des Elastomeren und erfordern daher Beschränkungen im Hinblick auf die optimale Gestaltung des Fertigungsprozesses (siehe hierzu ebenfalls A. V. Chapman, M. Porter in dem oben zitierten Artikel).

Ein Versuch zur Verringerung der Folgereaktionen des Schwefels stellt die Anwendung sogenannter EV-Systeme dar; dies sind Systeme, die aus erhöhten Mengen Vulkanisationsbeschleuniger bestehen bei gleichzeitig minimalen Mengen an Schwefel. Infolge der rascheren Anvulkanisation wird die Verarbeitungssicherheit jedoch nachteilig verändert. Dazu kommt - und dies ist von noch größerer Tragweite - die vorprogrammierte Beeinträchtigung des Abriebwiderstandes, der Weiterreißfestigkeit und der Kordhaftung gerade im Hinblick auf die Reifentechnologie (vgl. P. M. Lewis "Vulkanisate structure and its effects on properties" in NR-Technologie, Quaterly Volume 17, Part 4, Seite 57 ff., 1986).

Im Zusammenhang mit thermisch stabilen Vulkanisaten wurden Polysulfidderivate vorgeschlagen (vgl. DE-AS 22 65 382), die bei Zuführung thermischer Energie stabile Netzbrückenelemente freisetzen und als Abgangsgruppen (A) konventionelle Beschleunigersysteme z. B. Dithiocarbamoylstrukturen enthalten. Ein erheblicher Nachteil des Dithiocarbamatsystems liegt jedoch darin, daß im Laufe des Vulkanisationsprozesses Nitrosamine gebildet werden, die durch Nitrosierung der Zersetzungsprodukte (Amine) der unter diesen Bedingungen instabilen Dithiocarbamate entstehen. Nitrosamine müssen nach heutiger Erkenntnis als Gesundheitsrisiko angesehen werden, da sie zum großen Teil cancerogene Metaboliten bilden (vgl. Umschau 1985 (1), 24). Aminfreie Polysulfidderivate sind als Vernetzer ebenfalls bekannt, zeigen jedoch ein völlig unzureichendes Leistungsniveau.

Ziel der Erfindung war es, Vernetzungssysteme für Dienkautschuke zu schaffen, die die Reversion verringern, insbesondere den Leistungsabfall bei der Alterung der Elastomere bremsen und gleichzeitig die Gefahr cancerogener Nitrosamine umgehen.

Es wurden jetzt solche Vernetzungssysteme gefunden, mit denen dieses Ziel erreichbar ist. Der erfinderische Gedanke besteht darin, daß anstelle oligomerer S-Brücken zur Netzwerkformierung stabile kohlenstoffhaltige Brücken (X) mit aminfreien phenolischen Thiocarbonylabgangsgruppen gemäß folgender allgemeinen Formel kombiniert werden:

$(A - S - S -)_z X - S - S - A'$

A und A' sind dabei verschiedene oder gleichartige aminfreie aromatische Thiocarbonylgruppen der allgemeinen Formel

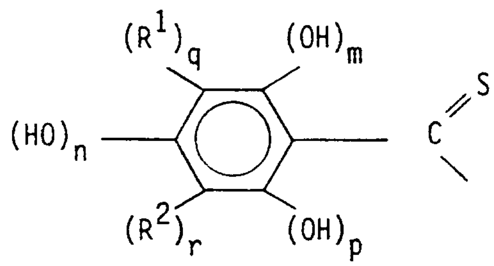

n, m, p, q und r haben die Bedeutung von 0 oder 1 mit der Einschränkung, daß die Summe n + m + p mindestens den Wert 1 haben muß.

$R^1$ und $R^2$ sind gleiche oder unterschiedliche Reste folgender Bedeutung
- Alkylrest mit 1 bis 10 C-Atomen
- Alkenylrest mit 1 bis 10 C-Atomen
- Aralkylrest mit bis zu 15 C-Atomen

Vorteilhafterweise können die phenolischen Gruppen auch in Form ihrer Alkali-, Erdalkali- oder Zinksalze vorliegen,

z ist eine natürliche Zahl mit $0 < z < 50$,

X hat die Bedeutung von zweiwertigen (z = 1) oder mehrwertigen (z>1) linearen oder verzweigten Aliphaten, Cycloaliphaten oder Araliphaten, wobei hierunter auch Polymere bis zu einem Polymerisationsgrad von 50 und einer Wertigkeit von mindestens zwei verstanden werden.

Für z = 1 hat X folgende Bedeutung:
- Alkylrest mit 1 bis 30 C-Atomen
- Alkenylrest mit 1 bis 30 C-Atomen
- Aralkylrest mit bis zu 40 C-Atomen.

Für $1 < z \leqq 4$ hat X folgende Bedeutung:
- Alkylrest mit 1 bis 20z C-Atomen
- Alkenylrest mit 1 bis 20z C-Atomen
- Aralkylrest mit bis zu 20z C-Atomen.

Bevorzugte Abgangsgruppen A, A' sind 4-Hydroxy-3,5-dialkyl-benzol-thiocarbonylgruppen, wobei gegebenenfalls statt der Phenolgruppe auch eine Phenolatgruppe von Vorteil ist. Besonders bevorzugt ist die 4-Hydroxy-3,5-di-tert.-butyl-benzol-thiocarbonyl-Abgangsgruppe bzw. das entsprechende Phenolat.

Für die Vernetzerbrücken X wird die Vielzahl der möglichen Verbindungen weiterhin durch die Verfügbarkeit der Ausgangsverbindungen der Dichloride und der Dithiole eingeschränkt. Bevorzugte Vernetzerbrücken X sind zweiwertige lineare Alkylengruppen, wobei besonders bevorzugt die Ethylengruppe eingesetzt wird.

Für das erfindungsgemäße Vernetzungsverfahren kommen als Kautschuke Natur- und Synthesekautschuke infrage. Bevorzugte Synthesekautschuke sind z. B. Styrol-Butadien-Copolymere, Polybutadiene, Polyisoprene, Integralkautschuke (wie in DE-OS 37 10 002, 37 24 871, 38 04 547 beschrieben) oder deren Verschnitte, aber auch Nitril-, Chloroprenbutyl-EPDM-Kautschuke und andere.

Bezüglich weiterer üblicher Kautschukzusätze, wie z. B. Füllstoffe, Weichmacher, Klebrigmacher, Beschleuniger, Aktivatoren, Sterinsäure, Wachse, Alterungs- und Ozonschutzmittel, Treibmittel, Farbstoffe sowie Pigmente gibt es keine Einschränkungen. Die Herstellung eines Vulkanisiersystems mit o. a. Kautschukzusätzen erfolgt nach üblichen, dem Fachmann bekannten Verfahren.

Die Vulkanisation wird bei Temperaturen zwischen 100 und 250 °C, bevorzugt zwischen 120 und 240 °C, durchgeführt. Die Vulkanisation geschieht ebenfalls nach üblichen, dem Fachmann bekannten Verfahren.

Die erfindungsgemäßen Polysulfidderivate weisen in den zu verwendenden Rezepturen Vorteile hinsichtlich Reversionsstabilität nach längerer Vulkanisationszeit und -temperaturen auf. Gleichzeitig zeigen sie in der Elastomeralterung einen erheblich geringeren Leistungsabfall und sind unbedenklich im Hinblick auf die Gefahr der Entstehung cancerogener Nitrosamine. Sie vereinigen in sich eine Doppelfunktion aus Vernetzungsreagenz (Bis-thio-Alkanbrücken) mit Antioxidans (sterisch gehindertes Phenol), wobei letzteres noch den zusätzlichen Vorteil einer Fixierung im Netzwerk aufweist.

Weiterer Gegenstand der Erfindung ist ein Verfahren zur Herstellung o. a. Polysulfidderivate durch Reaktion einer Dithiocarbonsäure der Formel

A - SH ,

wobei A die o. a. Bedeutung haben soll, mit dem Buntesalz zwei- oder mehrwertigen Alkylverbindung der Formel

$(NaO_3SS-)_z$ X $-SSO_3Na$ ,

wobei X und z ebenfalls die o. a. Bedeutungen haben, dadurch gekennzeichnet, daß man 2 Mol der Dithiocarbonsäure als Lösung in einem mit Wasser mischbaren organischen Lösungsmittel zu einem Mol des Buntesalzes als wäßrige oder organische mit Wasser mischbare Lösung bei Temperaturen von -10 bis +60 °C, vorzugsweise 20 bis 40 °C, eintropft und den pH der Reaktionslösung und alle Edukte zwischen 6 und 9 konstant hält, vorzugsweise zwischen 7 und 8.

Bevorzugtes organisches Lösungsmittel ist Tetrahydrofuran (THF).

Herstellungsbeispiele

1. Buntesalz des 1,2-Dichlorethans

40,16 g 1,2-Dichlorethan (97,5%ig) werden mit 198,40 g Natriumthiosulfatpentahydratin einem Gemisch aus 800 ml Wasser und 650 ml Ethanol 15 h bei Rückfluß gekocht. Das entstandene Bis-Buntesalz wird ohne weitere Aufarbeitung für die nächsten Stufen eingesetzt.

2. DBCS: 4-Hydroxy-3,5-di-tert.-butyl-benzoldithiocarbonsäure bzw. DBCS-K: das entsprechende Kaliumsalz

In einen Stickstoff gespülten 6 l-Dreihalskolben mit Rückflußkühler, Tropftrichter und Rührer werden 1 031,7 g Di-tert.-butylphenol unter Stickstoff in 2,5 l-THF gelöst. Nach Eintragen von 561,0 g KOH-Pulver (exotherme Reaktion) wird der gesamte Kolbeninhalt auf 10 °C abgekühlt. Bei dieser Temperatur werden über 5 h 380,5 g Schwefelkohlenstoff eingetropft und anschließend 15 h bei Temperatur nachreagieren lassen. Der entstandene Feststoff wird abgesaugt, mit kaltem Aceton nachgewaschen und im Vakuumtrokkenschrank bei Raumtemperatur getrocknet. Es lagen 1 525 g des Rohproduktes von GBCS-K vor (85 % Ausbeute).

3. DBEB: 1,2-Bis-(4-hydroxy-3,5-di-tert.-butyl-benzolthiocarbonyldithio-ethan
(Korrekter Name gemäß IUPAC: Bis-[4-hydroxy-3,5-di-tert.-butyl-benzol-dithiocarbonsäure-(dithioperoxo)]-1,2-ethandiylester)

294,1 g DBCS-K (aus 2.) als 10%ige wäßrige Lösung werden mit 1 200 ml THF versetzt und mit Essigsäure auf pH 7 eingestellt. Zu dieser Vorlage tropft man einerseits 1 516,8 g der Buntesalzlösung aus 1. (entspricht 0,4 Mol Buntesalz) und parallel dazu 67,3 g einer 37%igen Formaldehydlösung über einen Zeitraum von 3 h. Anschließend stellt man den pH auf 8 ein und läßt 15 h bei Raumtemperatur sowie 5 h bei 40 °C nachreagieren. Es lassen sich 171 g Rohprodukt isolieren, die aus Petrolether umkristallisierbar sind.

| Elementaranalyse: | C | H | O | S |
|---|---|---|---|---|
| berechnet | 58,67 | 7,08 | 4,88 | 29,36 |
| gefunden | 58,37 | 7,15 | | 27 |

Charakteristische IR-Banden (in $cm^{-1}$):
3 600, 2 960, 1 580, 1 215
$^{13}C$-NMR-Spektrum (in $CDCl_3$):

| | | | | | |
|---|---|---|---|---|---|
| Peaks bei | 30,015,<br>125,295, | 34,277,<br>135,564, | 36,611,<br>136,036, | 76,570,<br>159,086, | 76,922<br>225,316 ppm. |

Vulkanisationsbeispiele

Experimenteller Teil

Sämtliche der nachfolgend beschriebenen Laufflächen-Vulkanisate wurden wie folgt hergestellt:

Mittels eines Innenmischers vom Typ GK 2 der Firma Werner und Pfleiderer wird bei einer Rotordrehzahl von 50 UpM sowie einer Manteltemperatur von 40 °C folgende Grundmischung bereitet:

| | |
|---|---|
| Kautschuk | 100 |
| ZnO | 3 phr |
| Stearinsäure | 2 phr |
| aromatisches Weichmacheröl | 10 phr |
| Ruß N-339 | 50 phr |
| Vernetzungsmittel | siehe Tabellen |

Der Spannungswert, auch Modul genannt, bei 100 bzw. 300 % Dehnung wurde gemäß DIN 53 504 bestimmt.
Die Strukturfestigkeit wurde nach Pohle (vgl. S. Boström, Kautschuk-Handbuch, Band 5, Seite 123) bestimmt.
Die bleibende Dehnung (Zugverformungsrest) wurde gemäß DIN 53 518 bestimmt.
Die Härte (Shore A) wurde gemäß DIN 53 505 bestimmt.
Die Rückprallelastizität (Elast.) wurde gemäß DIN 53 512 bestimmt.
Der Abrieb wurde gemäß DIN 53 516 bestimmt.
Der Druckverformungsrest (Compression set) wurde gemäß DIN 53 517 bestimmt.
Die Prüfung der Wärmebildung (heat built-up) erfolgt gemäß DIN 53 533, Teil 3, mit Hilfe des Goodrich-Flexometers, im Versuchsteil als Methode 1 bezeichnet. Darüber hinaus wurden folgende erschwerte Bedingungen gewählt, die im Versuchsteil als Methode 5 bezeichnet werden:
Last: 500 N, Starttemperatur: 50 °C, Zeit: 25 Minuten.

Die Kugelzermürbung bei den jeweiligen Belastungen wurde gemäß S. Baström, Kautschuk-Handbuch, 5. Band, Berliner Union, Stuttgart, S. 149, 150, bestimmt.
Die Vulkametrie wurde gemäß DIN 53 529 vorgenommen.

Beispiel 1

Vulkanisation eines vormastizierten NR (Mooney 70; RRS Nr. 3) mit DBEB (7,0 phr) bei erhöhter Reaktionstemperatur (1. Teil)

| Vulkanisationstemperatur | | | 150 °C | | | Beispiel 180 °C | | |
|---|---|---|---|---|---|---|---|---|
| Vulkanisationszeit in min. | | | 30 | 5 | 10 | 15 | 20 | 30 |
| Vulkameter 150 °C | | | | | | | | |
| $t_{10}$ | | min | 5,7 | | | | | |
| $t_{90}$ | | min | 12,1 | | | | | |
| Delta t | | min | 6,4 | | | | | |
| Zugfestigkeit | | MPa | 21,6 | 20,8 | 19,1 | 18,9 | 18,0 | 17,2 |
| Bruchdehnung | | % | 581 | 528 | 498 | 476 | 468 | 445 |
| Modul | 100 % | MPa | 1,4 | 1,5 | 1,6 | 1,6 | 1,6 | 1,5 |
| | 300 % | MPa | 8,3 | 8,8 | 9,1 | 9,2 | 9,3 | 9,1 |
| Strukturfestigkeit | | N/mm | 81 | 84 | 78 | 72 | 72 | 69 |
| bleibende Dehnung | | % | 10 | 10 | 10 | 11 | 10 | 11 |
| Härte | 22 °C | Sh.A | 62 | 62 | 61 | 62 | 62 | 61 |
| | 75 °C | Sh.A | 58 | 54 | 54 | 53 | 54 | 53 |
| Elastizität | 22 °C | % | 47 | 46 | 47 | 47 | 47 | 46 |
| | 75 °C | % | 58 | 57 | 56 | 56 | 56 | 56 |
| DIN-Abrieb | | $mm^3$ | 133 | 125 | 123 | 131 | 135 | 134 |
| spez. Gewicht | | $g/mm^3$ | 1,10 | 1,10 | 1,10 | 1,10 | 1,10 | 1,10 |

Vergleichsbeispiel 1

Vulkanisation eines vormastizierten NR (Mooney 70; RRS Nr. 3) mit dem Vulkanisationssystem Schwefel/CBS (2,5/0,6 phr) bei erhöhter Reaktionstemperatur

| Vulkanisationstemperatur | | | 150 °C | | | Beispiel 180 °C | | |
|---|---|---|---|---|---|---|---|---|
| Vulkanisationszeit in min. | | | 30 | 5 | 10 | 15 | 20 | 30 |
| Vulkameter 150 °C | | | | | | | | |
| $t_{10}$ | | min | 7,6 | | | | | |
| $t_{90}$ | | min | 12,5 | | | | | |
| Delta t | | min | 4,9 | | | | | |
| Zugfestigkeit | | MPa | 23,3 | 18,8 | 17,5 | 14,5 | 13,8 | 11,9 |
| Bruchdehnung | | % | 558 | 498 | 424 | 510 | 526 | 483 |
| Modul | 100 % | MPa | 2,0 | 1,6 | 1,3 | 1,3 | 1,1 | 1,1 |
| | 300 % | MPa | 10,1 | 8,5 | 6,6 | 5,7 | 5,4 | 5,3 |
| Strukturfestigkeit | | N/mm | 74 | 64 | 26 | 19 | 18 | 16 |
| bleibende Dehnung | | % | 16 | 15 | 13 | 9 | 11 | 10 |
| Härte | 22 °C | Sh.A | 61 | 58 | 55 | 54 | 53 | 52 |
| | 75 °C | Sh.A | 57 | 52 | 46 | 45 | 44 | 43 |
| Elastizität | 22 °C | % | 48 | 50 | 47 | 45 | 46 | 44 |
| | 75 °C | % | 60 | 60 | 54 | 51 | 50 | 49 |
| DIN-Abrieb | | $mm^3$ | 129 | 121 | 148 | 189 | 224 | 247 |
| spez. Gewicht | | $g/mm^3$ | 1,10 | 1,10 | 1,10 | 1,10 | 1,10 | 1,10 |

Beispiel 2

Vulkanisation eines SBR 1 500 mit DBEB

| Vulkanisationssystem | | 7 phr DBEB | | | | |
|---|---|---|---|---|---|---|
| Eigenschaften | | Vulkanisation bei 150 °C in 40 Min. | nach 7 d Alterung bei 100 °C | 110 °C | 120 °C | 130 °C |
| Zugfestigkeit | MPa | 20,9 | 16,2 | 15,9 | 13,2 | 5,8 |
| Bruchdehnung | % | 478 | 278 | 247 | 181 | 58 |
| Modul 100 % | MPa | 2,2 | 3,6 | 4,1 | 5,1 | - |
| 300 % | MPa | 10,6 | - | - | - | - |
| Strukturfestigkeit | N/mm | 28 | 26 | 16 | 9 | - |
| bleibende Dehnung | % | 9 | 4 | 3 | 2 | 1 |
| Härte 22 °C | Sh.A | 64 | 68 | 71 | 73 | 76 |
| 75 °C | Sh.A | 60 | 63 | 68 | 69 | 73 |
| Elastizität 22 °C | % | 45 | 52 | 50 | 53 | 54 |
| 75 °C | % | 60 | 62 | 63 | 62 | 66 |
| DIN-Abrieb | $mm^3$ | 95 | 120 | 129 | 138 | 158 |
| spez. Gewicht | $g/mm^3$ | 1,12 | 1,12 | 1,12 | 1,12 | 1,12 |
| Compr. Set 24 h/70 °C | % | 17 | 13 | 12 | 13 | 12 |

Vergleichsbeispiel 2

Vulkanisation eines SBR 1 500 mit Schwefel/CBS

Vulkanisationssystem Schwefel/CBS = 2,2/1,2 phr

| Eigenschaften | | Vulkanisation bei 150 °C in 40 Min. | nach 7 d Alterung bei 100 °C | 110 °C | 120 °C | 130 °C |
|---|---|---|---|---|---|---|
| Zugfestigkeit | MPa | 22,0 | 18,3 | 9,5 | 6,2 | 3,6 |
| Bruchdehnung | % | 427 | 309 | 124 | 35 | - |
| Modul 100 % | MPa | 2,4 | 3,5 | 7,1 | - | - |
| 300 % | MPa | 13,1 | 17,7 | - | - | - |
| Strukturfestigkeit | N/mm | 31 | 26 | 6,8 | - | - |
| bleibende Dehnung | % | 8 | 5 | 2 | 1 | 1 |
| Härte 22 °C | Sh.A | 66 | 73 | 78 | 82 | 87 |
| 75 °C | Sh.A | 60 | 69 | 75 | 78 | 85 |
| Elastizität 22 °C | % | 44 | 60 | 57 | 41 | 38 |
| 75 °C | % | 68 | 65 | 63 | 56 | 53 |
| DIN-Abrieb | $mm^3$ | 108 | 118 | 159 | 167 | 220 |
| spez. Gewicht | $g/mm^3$ | 1,12 | 1,12 | 1,12 | 1,12 | 1,12 |
| Compr. Set 24 h/70 °C | % | 14 | 11 | 12 | 13 | 13 |

**Patentansprüche**

**1.** Sulfidderivate aormatischer Dithiocarbonsäuren der Formel

(A - S - S -$)_z$ X - S - S - A',

wobei A und A' verschiedene oder gleichartige aminfreie aromatische Thiocarbonylgruppen der allgemeinen Formel

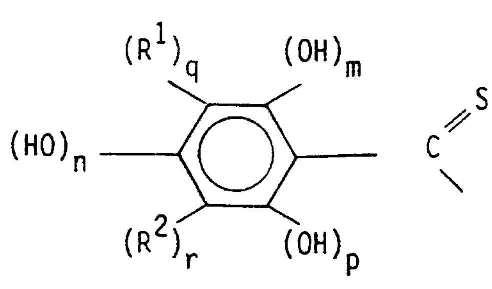

sind,

n, m, p, q und r haben die Bedeutung von 0 oder 1 mit der Einschränkung, daß die Summe n + m + p mindestens den Wert 1 haben muß,

$R^1$ und $R^2$ sind gleiche oder unterschiedliche Reste folgender Bedeutung

- Alkylrest mit 1 bis 10 C-Atomen

- Alkenylrest mit 1 bis 10 C-Atomen
- Aralkylrest mit bis zu 15 C-Atomen,

z ist eine natürliche Zahl mit $0 < z < 50$,
X hat die Bedeutung von zweiwertigen ($z = 1$) oder mehrwertigen ($z > 1$) linearen oder verzweigten Aliphaten, Cycloaliphaten oder Araliphaten, wobei hierunter auch Polymere bis zu einem Polymerisationsgrad von 50 und einer Wertigkeit von mindestens zwei verstanden werden,
für $z = 1$ hat X folgende Bedeutung:

- Alkylrest mit 1 bis 30 C-Atomen
- Alkenylrest mit 1 bis 30 C-Atomen
- Aralkylrest mit bis zu 40 C-Atomen,

für $1 < z \leqq 4$ hat X folgende Bedeutung:

- Alkylrest mit 1 bis 20z C-Atomen
- Alkenylrest mit 1 bis 20z C-Atomen
- Aralkylrest mit bis zu 20z C-Atomen.

**2.** Verbindung gemäß Anspruch 1,
dadurch gekennzeichnet,
dar A bzw. A' 4-Hydroxy-3,5-dialkyl-benzol-thiocarbonylgruppen bzw. die entsprechenden Phenolate sind.

**3.** Verbindung gemäß Anspruch 1,
dadurch gekennzeichnet,
daß X eine Ethylengruppe darstellt.

**4.** Verfahren zur Herstellung der Substanz nach Anspruch 1,
dadurch gekennzeichnet,
daß Dithiocarbonsäuren der Formel

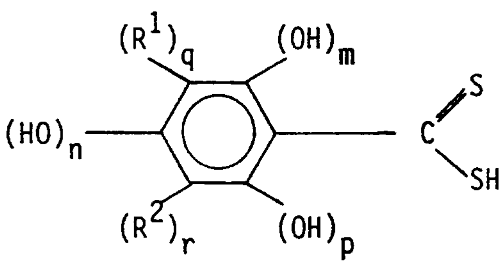

wobei n, m, p, q und r die Bedeutung von 0 oder 1 haben soll, mit der Bedingung, daß n + m + q mindestens 1 sein muß und $R^1$ und $R^2$ gleiche oder unterschiedliche Rest folgender Bedeutung sind:

- Alkylrest mit 1 bis 10 C-Atomen
- Alkenylrest mit 1 bis 10 C-Atomen
- Aralkylrest mit bis zu 15 C-Atomen,

mit dem Buntesalz zwei- oder mehrwertiger Alkylverbindungen der Formel

$(NaO_3SS)_z X - SSO_3Na$

wobei z eine natürliche Zahl zwischen 0 und 50 darstellt,
x die Bedeutung von zweiwertigen ($z = 1$) oder mehrwertigen ($z > 1$) linearen oder verzweigten Aliphaten, Cycloaliphaten oder Araliphaten hat, wobei hierunter auch Polymere bis zu einem Polymerisationsgrad von 50 und einer Wertigkeit von mindestens zwei verstanden werden,
für $z = 1$ hat X folgende Bedeutung:

- Alkylrest mit 1 bis 30 C-Atomen
- Alkenylrest mit 1 bis 30 C-Atomen
- Aralyklrest mit bis zu 40 C-Atomen

für $1 < z \leqq 4$ hat X folgende Bedeutung:

- Alkylrest mit 1 bis 20z C-Atomen
- Alkenylrst mit 1 bis 20z C-Atomen

- Aralkylrest mit bis zu 20z C-Atomen

bei Temperaturen von -10 bis +60 °C und pH-Werten zwischen 6 und 9 reagieren läßt.

**5.** Verwendung der Substanz gemäß der Ansprüche 1 bis 3 zur Vernetzung von Natur- und Synthesekautschuken.

## Claims

**1.** A sulphide derivative of an aromatic dithiocarboxylic acid of the formula

$(A - S - S -)_z X - S - S - A'$,

where A and A' are identical or different amine-free aromatic thiocarbonyl groups of the general formula

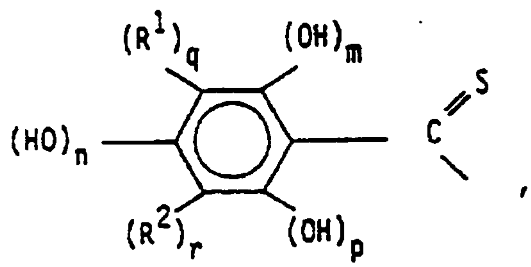

n, m, p, q and r are 0 or 1, with the restriction that the sum n + m + p must be at least 1,
$R^1$ and $R^2$ are identical or different radicals of the following meaning:

- alkyl radical having 1 to 10 carbon atoms,
- alkenyl radical having 1 to 10 carbon atoms,
- aralkyl radical having up to 15 carbon atoms,

z is a natural number where $0 < z < 50$,
X is a divalent (z = 1) or polyvalent (z > 1) linear or branched aliphatic, cycloaliphatic or araliphatic radical, including polymers having a degree of polymerization of up to 50 and a valency of at least two,
for z = 1, X has the following meaning:

- alkyl radical having 1 to 30 carbon atoms,
- alkenyl radical having 1 to 30 carbon atoms
- aralkyl radical having up to 40 carbon atoms,

for $1 < z \leq 4$, X has the following meaning:

- alkyl radical having 1 to 20z carbon atoms,
- alkenyl radical having 1 to 20z carbon atoms,
- aralkyl radical having up to 20z carbon atoms.

**2.** A compound according to claim 1, characterized in that A and A' are 4-hydroxy-3,5-dialkylbenzenethiocarbonyl groups or the corresponding phenoxides.

**3.** A compound according to claim 1, characterized in that X is an ethylene group.

**4.** A process for the preparation of a substance according to claim 1, characterized in that a dithiocarboxylic acid of the formula

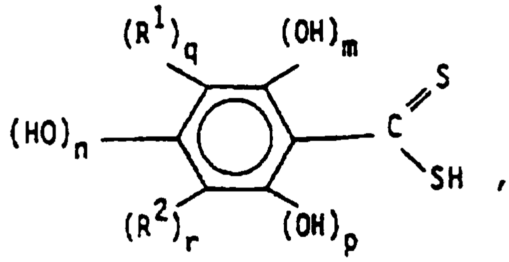

where n, m, p, q and r are 0 or 1, with the proviso that n + m + q must be at least 1, and $R^1$ and $R^2$ are identical or different radicals of the following meaning:

- alkyl radical having 1 to 10 carbon atoms,
- alkenyl radical having 1 to 10 carbon atoms,
- aralkyl radical having up to 15 carbon atoms,

is allowed to react with the Bunte salt of a divalent or polyvalent alkyl compound, of the formula

$(NaO_3SS)_z X - SSO_3Na$,

where z is a natural number from 0 to 50,
X is a divalent (z = 1) or polyvalent (z > 1) linear or branched aliphatic, cycloaliphatic or araliphatic radical, including polymers having a degree of polymerization of up to 50 and a valency of at least two,
for z = 1, X has the following meaning:

- alkyl radical having 1 to 30 carbon atoms,
- alkenyl radical having 1 to 30 carbon atoms,
- aralkyl radical having up to 40 carbon atoms,

for $1 < z \leqq 4$, X has the following meaning:

- alkyl radical having 1 to 20z carbon atoms,
- alkenyl radical having 1 to 20z carbon atoms,
- aralkyl radical having up to 20z carbon atoms,

at a temperature of from -10 to +60 °C and at a pH of from 6 to 9.

**5.** The use of a substance according to any claims 1 to 3 for crosslinking natural and synthetic rubbers.

## Revendications

**1.** Dérivés sulfurés d'acides dithio-carboxyliques aromatiques de formule

$(A - S - S -)_z X - S - S - A'$,

dans laquelle A et A' sont des groupes thio-carbonyle aromatiques identiques ou différents, exempts d'amine et répondant à la formule générale

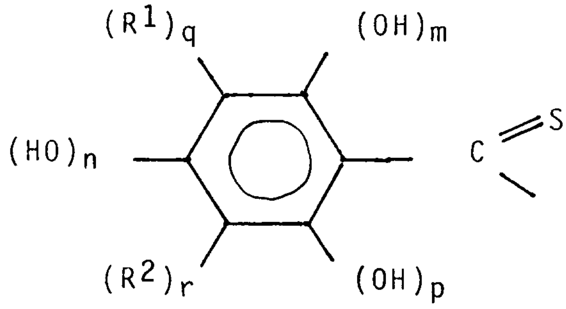

n, m, p, q et r ont la signification de 0 ou 1 avec cette restriction que la somme n + m + p doit avoir

au moins la valeur 1,
$R_1$ et $R_2$ sont des radicaux identiques ou différents ayant la signification suivante :

- radical alkyle comportant de 1 à 10 atomes de carbone,
- radical alkényle comportant de 1 à 10 atomes de carbone,
- radical aralkyle comportant jusqu'à 15 atomes de carbone,

z est un nombre naturel répondant à l'inégalité $0 < z < 50$,
X représente des radicaux aliphatiques cyclo-aliphatiques ou araliphtatiques, linéaires ou ramifiés, bivalents (z = 1) ou polyvalents (z>1), parmi lesquels il y a lieu aussi d'entendre des polymères d'un degré de polymérisation allant jusqu'à 50 et une valence de deux au moins,
tandis que pour z = 1 X a la signification suivante :

- radical alkyle conportant de 1 à 30 atomes de carbone,
- radical alkényle comportant de 1 à 30 atomes de carbone,
- radical aralkyle comportant jusqu'à 40 atomes de carbone,

et que pour la relation $1 < z \leqq 4$, X a la signification suivante :

- radical alkyle comportant de 1 à 20z atomes de carbone,
- radical alkényle comportant de 1 à 20z atomes de carbone,
- radical aralkyle comportant jusqu'à 20z atomes de carbone.

**2.** Composé selon la revendication 1,
caractérisé par le fait que A et A' sont des groupes 4-hydroxy-3,5-dialkyl-benzène-thio-carbonyle ou les phénolates correspondants.

**3.** Composé selon la revendication 1,
caractérisé par le fait que X représente un groupe éthylène.

**4.** Procédé de préparation de la substance selon la revendication 1,
caractérisé par le fait que l'on fait réagir à des températures de -10 à + 60° et à des valeurs du pH comprises entre 6 et 9, des acides dithio-carboxyliques de formule

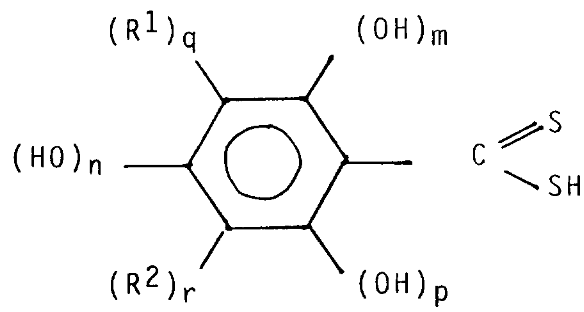

n, m, p, q et r ayant la signification de 0 ou 1 avec cette condition que la somme n + m + q doit avoir au moins la valeur 1, et $R_1$ et $R_2$ étant des radicaux identiques ou différents ayant la signification suivante :

- radical alkyle comportant de 1 à 10 atomes de carbone,
- radical alkényle comportant de 1 à 10 atomes de carbone,
- radical aralkyle comportant jusqu'à 15 atomes de carbone,

sur le poly-sel d'alkyl-composés bivalents ou polyvalents de formule

$(NaO_3SS)_z X - SSO_3Na$

z est un nombre naturel compris entre 0 et 50,
x représente des radicaux aliphatiques, cyclo-aliphatiques ou araliphatiques, linéaires ou ramifiés, bivalents (z = 1) ou polyvalents (z>1), parmi lesquels il y a lieu d'entendre aussi des polymères d'un degré de polymérisation allant jusqu'à 50 et d'une valence d'au moins deux,
tandis que pour z = 1, X a la signification suivante :

- radical alkyle comportant de 1 à 30 atomes de carbone,
- radical alkényle comportant de 1 à 30 atomes de carbone,

- radical aralkyle comportant jusqu'à 40 atomes de carbone,

et que pour la relation $1 < z \leqq 4$, X a la signification suivante :

- radical alkyle comportant de 1 à 20z atones de carbone,
- radical alkényle comportant de 1 à 20z atomes de carbone,
- radical aralkyle comportant jusqu'à 20z atomes de carbone.

**5.** L'utilisation de la substance selon les revendications 1 à 3, pour réticuler des caoutchoucs naturels et des caoutchoucs de synthèse.